(19) Europäisches Patentamt
European Patent Office
Office européen des brevets

(11) Publication number: 0 262 876 B1

(12) **EUROPEAN PATENT SPECIFICATION**

(45) Date of publication of patent specification: **29.04.92**

(51) Int. Cl.[5]: **C07H 19/10**, C07D 473/34, A61K 31/70, A61K 31/52

(21) Application number: **87308497.4**

(22) Date of filing: **25.09.87**

(54) **Nucleoside-phospholipid conjugate.**

(30) Priority: **27.09.86 JP 229203/86**
**27.09.86 JP 229204/86**

(43) Date of publication of application:
**06.04.88 Bulletin 88/14**

(45) Publication of the grant of the patent:
**29.04.92 Bulletin 92/18**

(84) Designated Contracting States:
**DE FR GB**

(56) References cited:
**EP-A- 0 122 151**
**US-A- 4 471 113**

**JOURNAL OF MEDICINAL CHEMISTRY, vol. 25, 1982, pages 1322-1329, American Chemical Society, Columbus, Ohio, US; E.K. RYU et al.: "Phospholipid-nucleoside conjugates. 3.' Syntheses and preliminary biological evaluation of 1-beta-D-arabinofuranosylcytosine 5'-monophosphate-L-1,2-dipalmitin and selected 1-beta-D-arabinofuranosylcytosine 5'-diphosphate-L-1,2-diacylglycerols"**

(73) Proprietor: **TOYO JOZO KABUSHIKI KAISHA**
**632-1 Mifuku Ohito-cho**
**Tagata-gun Shizuoka-ken(JP)**

(72) Inventor: **Shuto, Satoshi**
**Coop Watanabe 201 48-6, Shintani Mishima-shi**
**Mishima-shi Shizuoka-ken(JP)**
Inventor: **Itoh, Hiromichi**
**379-15, Fujishiro-machi**
**Mishima-shi Shizuoka-ken(JP)**
Inventor: **Fukukawa, Kiyofumi**
**1477-12, Yada**
**Mishima-shi Shizuoka-ken(JP)**
Inventor: **Tujino, Masatoshi**
**375-4, Takyo Ohito-machi Tagata-gun**
**Shizuoka-ken(JP)**

(74) Representative: **Woods, Geoffrey Corlett et al**
**J.A. KEMP & CO. 14 South Square Gray's Inn**
**London WC1R 5EU(GB)**

EP 0 262 876 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 98, 1983, page 362, abstract no. 132201z, Columbus, Ohio, US; M. MacCOSS et al.: "Synthesis and biological activity of novel nucleoside-phospholipid prodrugs", & PROC. INT. ROUND TABLE NUCLEOSIDES, NUCLEOTIDES THEIR BIOL. APPL., 4TH 1981 (PUB. 1982), 255-63

TETRAHEDRON LETTERS, vol. 28, no. 2, 1987, pages 199-202, Pergamon Journals Ltd, Oxford, GB; S. SHUTO et al.: "A facile one-step synthesis of 5'-phosphatidiylnucleosides by an enzymatic two-phase reaction"

## Description

This invention relates to nucleoside-phospholipid conjugates and salts thereof.

Nucleoside antitumor agents have been widely used as effective chemotherapeutics for neoplastic cells. In their application as antitumor-chemotherapeutics, however, several problems have been identified. For example, in the mechanism for the activity of nucleoside anti-neoplastic agents, phosphorylation of the hydroxyl group at position-5' of the nucleoside in vivo is essential for antitumor activity; the compound is decomposed to an inactive substance by, for example, phosphorolysis or deamination; the resistance of tumor cells to antitumor agents is increased; and the agent is sometimes toxic to mitotic normal cells. Many nucleoside derivatives have been synthesized to try to overcome the disadvantages of these nucleoside antitumor agents.

CDP-diacylglycerol is known to have an important role as an intermediate in the biosynthesis of glycerophospholipid in vivo, and its analogue, arabinosylcytosinephospholipid conjugate, which has antitumor activity, has been synthesized. [ Biochim. Biophys. Acta, 619, 619 - 631 ( 1980 ) and J. Med. Chem., 1982, 25, 1322 - 1329 ].

A 5-fluoro-2-deoxyuridine-phospholipid conjugate ( JP-A-61 - 9115 and JP-A-61 - 152694 ) and a process for manufacturing a heterocyclic phopholipid conjugate using phospholipase DM ( JP-A-61 - 88890 ) have also been reported.

The known compounds and processes for preparing them have a number of disadvantages. The synthesis of the compounds is a multi-step process with complicated operations and the overall yield is quite low. Furthermore the compounds are only slightly soluble in an aqueous vehicle and are difficult to use in injectable preparations.

A known nucleoside-phospholipid conjugate has a glyceride ester linkage and a hydrophobic fatty acid in a phospholipid skeleton, and hence is easily decomposed by phospholipase $A_1$ or phopholipase $A_2$ in vivo. The stability of the compound is also not satisfactory.

We have found that phospholipase D-P effectively catalyzes the transfer reaction of the phosphatidyl residue from a glycerophospholipid to the primary hydroxyl group of a nucleoside. Thus, a variety of nucleoside-phospholipid conjugates can be readily prepared.

We have also found nucleoside-phospholipid conjugates which have superior solubility in an aqueous medium and which have strong antitumor activity.

We have additionally found that ether type compounds such as nucleoside-dialkylether phospholipid conjugates or salts thereof are not decomposed by phospholipase $A_1$ or $A_2$ in vivo and have strong antitumor activity.

The present invention provides a nucleoside-phospholipid conjugate of the formula (I):

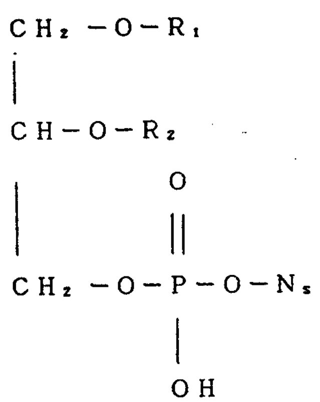

( I )

wherein $R_1$ is $C_{14-24}$ long chain aliphatic acyl, $C_{1-24}$ aliphatic alkyl, or $C_{2-24}$ aliphatic alkenyl or alkynyl, $R_2$ is $C_{1-10}$ aliphatic acyl, $C_{1-24}$ aliphatic alkyl, or $C_{2-24}$ aliphatic alkenyl or alkynyl, and Ns is a 5-fluorouridine-5'-yl, neoplanocin A-6'-yl or aribinosyl-5-fluorocytosine-5'-yl residue, and pharmacologically acceptable salts thereof.

The $C_{14-24}$ long-chain aliphatic acyl group represented by $R_1$ may be saturated or unsaturated. Examples of straight chain saturated aliphatic acyl groups are myristoyl, palmitoyl, stearoyl, eicosanoyl, dosaconoyl and tetracosanoyl groups. Examples of unsaturated aliphatic acyl groups are palmito-oleoyl, oleoyl and 9,12-octadecadienoyl groups. The $C_{1-24}$ aliphatic alkyl groups may be straight or branched;

examples are methyl, ethyl, propyl, butyl, hexyl, heptyl, 3-heptyl, octyl, nonyl, decyl, 8-ethyldecyl, undecyl, lauryl, tridecyl, pentadecyl, hexadecyl (cetyl), heptadecyl, octadecyl (stearyl), nonadecynol and eicodecyl groups. Examples of the $C_{2-24}$ aliphatic alkenyl groups are vinyl, allyl, 2-butenyl, 3-hexynyl, 4-decynyl, 6-tetradecenyl, 9-octadecenyl and linoleyl groups.

Examples of $C_{1-10}$ aliphatic acyl groups represented by $R_2$ are acetyl, propynoyl, butanoyl, hexanoyl, octanoyl and decanoyl groups. Examples of $C_{1-24}$ aliphatic alkyl or $C_{2-24}$ aliphatic alkenyl groups are the same as for $R_1$.

In the compound of formula (I), at least one of $R_1$ and $R_2$ is preferably a $C_{14-24}$ aliphatic alkyl, alkenyl or alkynyl group, for affinity to cell membranes in vivo.

Preferred conjugates of formula (I) are those wherein $R_1$ is $C_{16-18}$ long chain aliphatic acyl and $R_2$ is $C_{1-10}$ aliphatic acyl, $C_{1-10}$ aliphatic alkyl or aliphatic alkenyl or alkynyl containing up to 10 carbon atoms, or those wherein $R_1$ and $R_2$ are both $C_{1-24}$ aliphatic alkyl or $C_{2-24}$ aliphatic alkenyl or alkynyl.

Neplanocin A has the formula:

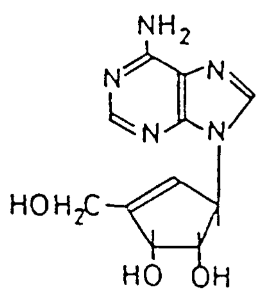

Pharmacologically acceptable salts of the compound of formula (I) are, for example, alkaline metal salts, alkaline earth metal salts, transition metal salts or organic base salts.

The present invention also provides a process for the preparation of a nucleoside-phospholipid conjugate of the formula (I) or a pharmacologically acceptable salt thereof, which process comprises reacting a glycerophospholipid derivative of formula (II):

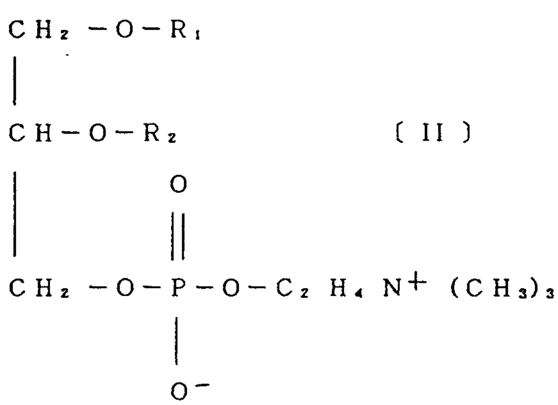

[ II ]

wherein $R_1$ and $R_2$ are as defined above, with a nucleoside of formula $N_s$-OH wherein $N_s$ is as defined above in a solvent in the presence of phospholipase D-P; and, if desired, converting the resultant compound of formula (I) into a pharmacologically acceptable salt thereof.

A preferred example of phospholipase D-P is phospholipase D-P obtained from Streptomyces sp. AA586 FERM P-6100 (JP-A-58-152481, Toyo Jozo Co., Catalogue No. P - 39 ). The amount of catalyst used is generally more than 0.01 unit phospholipase D-P per 0.001 mole of phospholipid of formula [II], and is preferably from 1 to 100 units. An example of a solvent which may be used is a two-phase system comprising an organic solvent and an aqueous solvent, for example a mixture of an organic solvent such as ether, benzene or chloroform and buffer solution of pH 3 to 9, preferably pH 4 to 6. An example of a water soluble salt for forming a metallic ion is calcium chloride. The reaction temperature is generally from 20 to

60 ° C and the reaction time is generally from 30 minutes to 30 hours, preferably from 1 to 6 hours. The thus-obtained nucleoside-phospholipid conjugate [ I ] can be purified, for example, by a partition method and silica-gel chromatography.

A one-step synthesis of a nucleoside-phospholipid conjugate of the present invention is illustrated as follows:

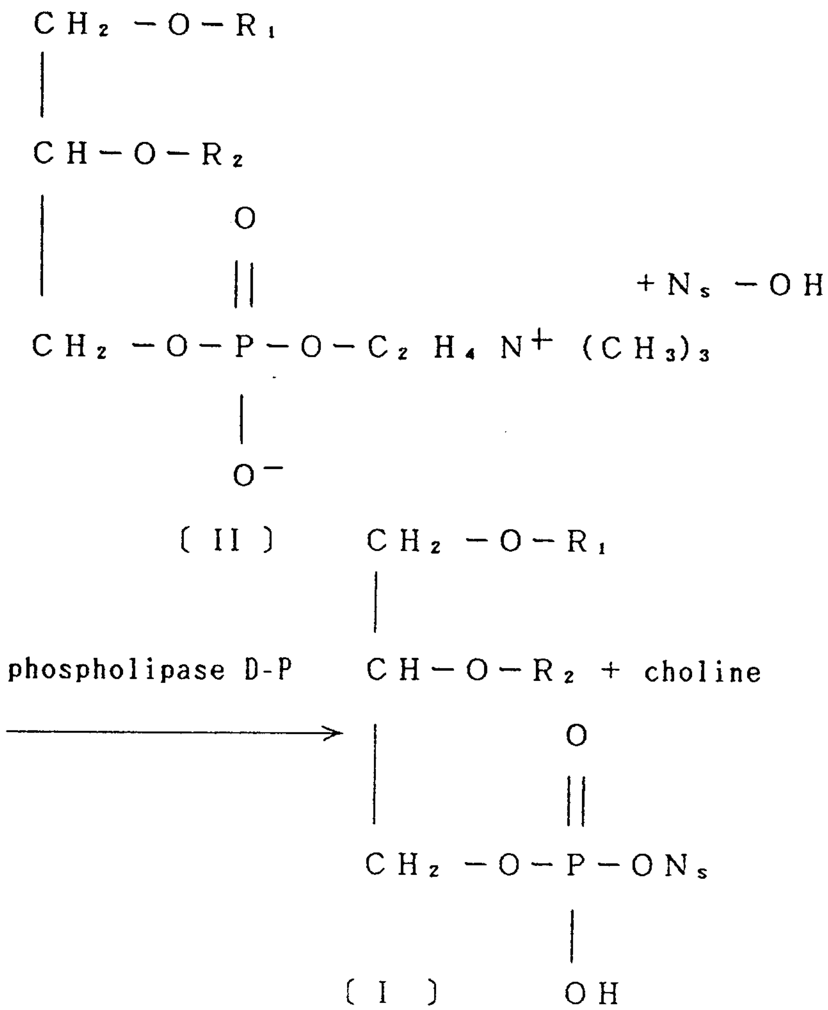


[ II ]

[ I ]

wherein $R_1$, $R_2$ and Ns are as defined above.

The thus obtained compound of formula (I) can be converted into a non-toxic, pharmacologically acceptable salt thereof such as a sodium or potassium salt.

The nucleoside-phospholipid conjugate of the present invention has the advantages that : it is more lipophilic as compared with the original nucleoside; it is not easily excreted, that is it is more active for a longer time; it is not affected by enzymatic inactivation such as phosphorolysis, deamination or reduction; it has higher affinity for cell membranes; an antineoplactic nucleoside 5'- monophosphate is generated in cells without the action of a kinase; and it has a long action and increased activity with low toxicity.

An ester type nucleoside-phospholipid conjugate of the present invention is especially resistant to hydrolysis by phospholipase $A_1$ or phospholipase $A_2$ in vivo and can be administered orally. The nucleoside-phospholipid conjugate of the present invention also has an advantage of high solubility in water and hence it can be prescribed not only in oral administration form but also as an injectable preparation.

The nucleoside-phospholipid conjugates of the present invention have marked antitumor activity in vivo, in addition to anti-metastatic activity of tumors and antiviral activity.

The antitumor activity against mouse leukemia P-388 carcinoma is now shown using the following specifications:

[ Antitumor activity ]

1. Samples : shown in Tables 1 and 2.
2. Animal : BDF, mice, aged 5 weeks, male, Charles River Inc., 5 mice in a group and 7 control mice.
3. Tumor cells : P-388 leukemic cells ; $1 \times 10^6$ / 0.2 ml are inoculated intraperitoneally in $BDF_1$ mice.
4. Preparation of samples and administration of drugs : : Samples are suspended in Tris-HCℓ saline solution by sonication. 0.1 ml / 10g body weight is administered.
Administration : starting one day after inoculation of tumor, once a day for 5 days intraperitoneally.
Amount of dose is shown in the Tables.
5. Increase in life span ( ILS ) is calculated by the following equation :

$$ILS(\%) = \frac{\text{mean increased life span (days); treated}}{\text{mean survivals date (days) ; control}} \times 100$$

The average survival time in the control group is 7.86 days ( 7.57 ~ 7.93 days ).

As shown in Table 1, the nucleoside-phospholipid conjugate illustrated in the Table is administered at 3 ~ 10 mg/kg/day in $BDF_1$ mice, and is shown to have a strong antitumor effect with an increased life span of 30 ~ 120 %.

Also as shown in Table 2, an ester type nucleoside-phosphlipid conjugate illustrated in the Table is administered at 10 ~ 30 mg/kg/day in $BDF_1$ mice, and is shown to have a strong antitumor activity with an increased life span of 30 ~ 150 %.

Table 1

| nucleoside-phospholipid conjugate | | | dose (mg/kg) | ILS (%) |
|---|---|---|---|---|
| $R_1$ | $R_2$ | $N_s$ | | |
| palmitoyl | decanoyl | FUR | 3 × 5<br>10 × 5 | 61.2<br>121.6 |
| " | octanoyl | FUR | 3 × 5<br>10 × 5 | 45.3<br>79.7 |
| " | hexanoyl | FUR | 3 × 5<br>10 × 5 | 45.3<br>79.7 |
| " | butanoyl | FUR | 3 × 5<br>10 × 5 | 36.1<br>53.2 |
| palmitoyl | acetyl | FUR | 3 × 5<br>10 × 5 | 40.1<br>58.9 |
| " | decanoyl | NEPA | 3 × 5<br>10 × 5 | 50.6<br>89.4 |
| " | octanoyl | NEPA | 3 × 5<br>10 × 5 | 66.4<br>84.9 |
| " | hexanoyl | NEPA | 3 × 5<br>10 × 5 | 34.7<br>53.2 |
| " | butanoyl | NEPA | 3 × 5<br>10 × 5 | 48.0<br>63.4 |
| " | acetyl | NEPA | 3 × 5<br>10 × 5 | 37.5<br>55.1 |
| FUR : 5 - fluorouridine 5'- yl<br>NEPA: neplanocin A - 6' - yl | | | | |

Table 2

| Ester type nucleoside-phospholipid conjugate | | | dose (mg/kg) | ILS (%) |
|---|---|---|---|---|
| $R_1$ | $R_2$ | $N_s$ | | |
| cetyl | cetyl | FUR | 10 × 5<br>30 × 5 | 101.0<br>127.0 |
| stearyl | stearyl | FUR | 30 × 5 | 31.0 |
| oleyl | oleyl | FUR | 10 × 5<br>30 × 5 | 74.0<br>76.0 |
| cetyl | cetyl | NepA | 30 × 5 | 34.9 |
| stearyl | stearyl | NepA | 30 × 5 | 31.0 |
| oleyl | oleyl | NepA | 30 × 5 | 26.0 |
| cetyl | cetyl | AraFC | 10 × 5<br>30 × 5 | 68.0<br>151.9 |
| stearyl | stearyl | AraFC | 30 × 5 | 61.0 |
| oleyl | oleyl | AraFC | 10 × 5<br>30 × 5 | 81.0<br>148.0 |
| FUR : 5 - fluorouridine - 5'-yl<br>NePA : neplanocin A - 6'- yl<br>AraFC: arabinosyl - 5 - fluorocytosine - 5'- yl | | | | |

[ Acute toxicity ]

Samples suspended in Tris-HCℓ saline solution by sonication shown in Tables 1 and 2 are administered intraperitoneally at doses of 100 mg/kg for the compounds shown in Table 1 or 200 mg/kg for the compounds shown in Table 2. No toxic signs are observed.

The present invention also provides a pharmaceutical composition comprising a nucleoside-phospholipid conjugate of formula (I) or a pharmacologically acceptable salt and a pharmacologically acceptable carrier or diluent.

The nucleoside-phospholipid conjugate of the present invention or salt thereof can be formulated by mixing with pharmacologically acceptable additives such as a carrier or diluent, as a tablet, capsule, granule, powder, injectable form or suppository, and can be administered orally or parenterally.

The daily dose is, for example, from 1 to 500 mg per man, with single or several doses. However, the dose depends on the age, body weight and symptoms and condition of the patient.

The following Examples further illustrate the present invention.

Examples 1 - 12

A nucleoside ($N_s$ - OH in an amount as shown in Table 3) was dissolved in 200 mM acetate buffer ( pH 6.0, is a volume as shown in Table 3 ) containing 250 mM $CaCℓ_2$ and stirred at 45°C for 3 mins. Phospholipase D-P ( 3 mg, from Streptomyces, Toyo Jozo Co. ) and a chloroform solution ( 10 ml ) of the phospholipid derivative [ II ] indicated in Table 3 ( 0.3 mM ) were added thereto and stirred for 6 hours, and then cooled. To the reaction mixture were added 1N - HCℓ ( 5 mℓ ), chloroform ( 30 ml ) and methanol ( 25 ml ); the reaction medium was then partitioned. The organic layer was washed twice with water and dried in vacuo. The residue was dried in vacuo after adding a small amount of ethanol, and dissolved in a small amount of chloroform, then charged on a flash column of silicagel ( 2.5 × 10 cm ) and eluted step-wise with chloroform, then a series of chloroform : methanol mixtures of relative concentrations ( 20 : 1 ), ( 15 : 1 ), ( 12 : 1 ), ( 10 : 1 ), ( 7 : 1 ), ( 5 : 1 ), ( 4 : 1 ) and ( 2 : 1 ), in this order. The eluate was dried in vacuo and the residue was dissolved in a mixture of chloroform ( 40 ml ) and methanol ( 20 ml ), separated by adding

0.5 N - HCℓ ( 12 ml ) and then washed twice with water and dried to yield the product.

Table 3

| No | $R_1$ | $R_2$ | $N_s$ − OH | Buffer solu. | Yield | PLDP | UV λmax | FAB mass spectrum | Rf |
|---|---|---|---|---|---|---|---|---|---|
| 1 | Palmitoyl | decanoyl | FUR, 10eq. | B', 3 ml | 59% | 3mg | 268nm | 831 (M+Na) | 0. 32 |
| 2 | 〃 | 〃 | NepA, 5eq. | B', 5ml | 63% | 3mg | 260nm | 832 (M+Na) | 0. 28 |
| 3 | 〃 | octanoyl | FUR, 10eq. | B', 3ml | 58% | 3mg | 268nm | 825 (M+2Na−H) | 0. 31 |
| 4 | 〃 | 〃 | NepA, 5eq. | B', 5ml | 61% | 3mg | 260nm | 826 (M+2Na−H) | 0. 25 |
| 5 | 〃 | hexanoyl | FUR, 10eq. | B', 3ml | 69% | 3mg | 268nm | 797 (M+2Na−H) | 0. 29 |
| 6 | 〃 | 〃 | NepA, 5eq. | B', 5ml | 73% | 3mg | 260nm | 776 (M+Na) | 0. 23 |
| 7 | 〃 | butanoyl | FUR, 10eq. | B', 3ml | 68% | 3mg | 268nm | 769 (M+2Na−H) | 0. 26 |
| 8 | 〃 | 〃 | NepA, 5eq. | B', 5ml | 72% | 3mg | 260nm | 748 (M+Na) | 0. 22 |
| 9 | 〃 | acetyl | FUR, 10eq. | B', 3ml | 65% | 3mg | 268nm | 741 (M+2Na−H) | 0. 21 |
| 10 | 〃 | 〃 | NepA, 5eq. | B', 5ml | 69% | 3mg | 260nm | 742 (M+2Na−H) | 0. 17 |
| 11 | stearoyl | octyl | NepA, 5eq. | B', 5ml | 62% | 3mg | 260nm | 818 (M+Na) | 0. 27 |

( contined table 3 )

| | | | | | | | | | |
|---|---|---|---|---|---|---|---|---|---|
| 12 | 〃 | 〃 | FUR. 20eq. | B'. 5ml | 55% | 3mg | 268nm | 719 (M+Na) | 0.22 |

$B_1$ : 200 mM acetate buffer ( pH 6.0 ) containing 250 mM $CaCl_2$

PLDP : Phospholipase D-P ( specific activity : 160 units / mg )

UV : measured in chloroform-methanol ( 1 : 2 )

Rf : developer : chloroform : methanol : water ( 65 : 25 : 30 )

Plate ; Merck, Art 5715

Spot was detected by UV and molybden-blue reagent.

<u>Examples 13 - 22</u>

A nucleoside ( $N_s$ - OH in Table 4 ) was dissolved or suspended in 200 mM acetate buffer ( pH 6.0, in a volume as shown in Table 4 ) containing 250 mM $CaCl_2$ and stirred at 45 °C for 3 minutes. Phospholipase D-P ( in an amount as shown in Table 4 ) and a chloroform solution ( 10 ml ) of the phospholipid derivative [

II ] ( 0.3 mM, illustrated in Table 4 ) were added thereto and stirred for 6 hours, and then cooled. 1N-HCℓ ( 5 ml ), chloroform ( 30 ml ) and methanol ( 25 ml ) were added thereto and the organic layer was separated. The organic layer was washed twice with water and dried in vacuo. A small amount of ethanol was added to the residue and it was dried in vacuo. The residue was dissolved in a small amount of chloroform and charged on a flash column of silica gel ( 2.5 × 10 cm ) and eluted stepwise with chloroform, then with chloroform : methanol in ratios of ( 20 : 1 ), (15 : 1 ), ( 12 : 1 ), ( 10 : 1 ), ( 7 : 1 ), ( 5 : 1 ), ( 4 : 1 ) and ( 3 : 1 ), in this order. The eluate was dried in vacuo and dissolved in a mixture of chloroform ( 40 ml ) and methanol ( 20 ml ), then 0.5 N-HCℓ ( 12 ml ) was added to separate the organic layer, which was washed twice with water. The residue was dried in vacuo to obtain the desired product.

NMR ( $CDCℓ_3$ : $CD_3$ OD = 2 : 1 )

1 : δ ppm 7.85 ( d, 1H, $J_{6F}$ = 6.3H ), 5.89 ( bs, 1H ), 4.3 ~ 3.9 ( m, 7H ), 3.53 ( m, 7H ), 1.55 ( br, 4H ), 1.26 ( S, 52H ), 0.88 ( t, 6H )

2 : δ ppm 8.23 ( s, 1H ), 8.19 ( s, 1H ), 5.89 ( bs, 1H ), 5.52 ( m, 1H ), 4.72 ( m, 3H ), 4.28 ( m, 1H ) 4.02 ( m, 2h ), 3.59 ( m, 7H ), 1.55 ( br, 4H ), 1.27 ( s, 52H ), 0.88 ( t, 6H )

3 : δ ppm 8.22 ( d, 1H, $J_{6F}$ = 6.5H ), 6.11 ( bs, 1H ), 4.3 ~ 4.0 ( m, 7H ), 3.53 ( m, 7H ), 1.55 ( br, 4H ), 1.26 ( s, 52H ), 0.88 ( t, 6H )

Table 4

| No | $R_1$ | $R_2$ | $N_s$ - OH | Buffer solu. | Yield | PLDP | UV λmax | FAB mass spectrum | Rf |
|---|---|---|---|---|---|---|---|---|---|
| 1 | cetyl | cetyl | FUR, 20 eq. | 3 ml | 60% | 3 mg | 268 nm | 887 (M+NBa) | 0.33 |
| 2 | 〃 | 〃 | NePA, 5 eq. | 7 m | 73% | 3 mg | 260 nm | 866 (MH) | 0.30 |
| 3 | 〃 | 〃 | AraFC, 10 eq. | 3 ml | 36% | 3 mg | 283 nm | 886 (M+Na) | 0.24 |
| 4 | 9-octadecenyl | 9-octadecenyl | FUR, 20 eq. | 5 ml | 62% | 2 mg | 268 nm | 939 (M+Na) | 0.39 |
| 5 | 〃 | 〃 | NepA, 5 eq. | 7 ml | 78% | 2 mg | 260 nm | 940 (M+Na) | 0.34 |
| 6 | stearyl | octyl | NepA, 5 eq. | 7 ml | 59% | 5 mg | 260 nm | 804 (M+Na) | 0.25 |
| 7 | stearyl | methyl | FUR, 20 eq. | 5 ml | 51% | 5 mg | 268 nm | 705 (M+Na) | 0.21 |
| 8 | cetyl | methyl | FUR, 20 eq. | 5 ml | 55% | 5 mg | 268 nm | 677 (M+Na) | 0.20 |
| 9 | cetyl | hexyl | FUR, 20 eq. | 5 ml | 61% | 5 mg | 268 nm | 747 (M+Na) | 0.25 |

Abbreviations are the same as of in Table 3

## Claims

**1.** A nucleoside-phospholipid conjugate of the formula (I):

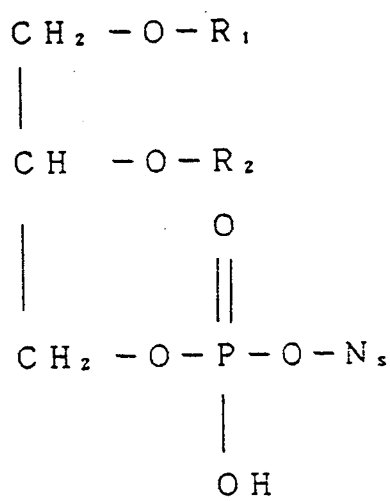

wherein $R_1$ is $C_{1-24}$ long-chain aliphatic acyl, $C_{1-24}$ aliphatic alkyl, or $C_{2-24}$ aliphatic alkenyl or alkynyl, $R_2$ is $C_{1-10}$ aliphatic acyl, $C_{1-24}$ aliphatic alkyl, or $C_{2-24}$ aliphatic alkenyl or alkynyl, and $N_s$ is a 5-fluorouridine-5'-yl, neplanocin A-6'-yl or aribinosyl-5-fluorocytosine-5'yl residue, and pharmacologically acceptable salts thereof.

**2.** A compound according to claim 1 wherein $R_1$ is $C_{16-18}$ long-chain aliphatic acyl and $R_2$ is $C_{1-10}$ aliphatic acyl.

**3.** A compound according to claim 1 wherein $R_1$ is $C_{16-18}$ long-chain aliphatic acyl and $R_2$ is $C_{1-10}$ aliphatic alkyl.

**4.** A compound according to claim 1 wherein $R_1$ and $R_2$ are both $C_{1-24}$ aliphatic alkyl.

**5.** A compound according to claim 1 wherein $R_1$ is palmitoyl, cetyl, stearyl, oleyl, stearoyl, or 9-octodecenyl and $R_2$ is decanoyl, octanoyl, hexanoyl, butanoyl, acetyl, cetyl, stearyl, oleyl, octyl, 9-octadecenyl, methyl or hexyl.

**6.** A compound according to claim 1 wherein the said nucleoside-phospholipid conjugate is an ether type compound.

**7.** A process for the preparation of a nucleoside-phospholipid conjugate of formula (I) as defined in claim 1 or a pharmacologically acceptable salt thereof, which process comprises reacting a glycerophospholipid derivative of formula (II):

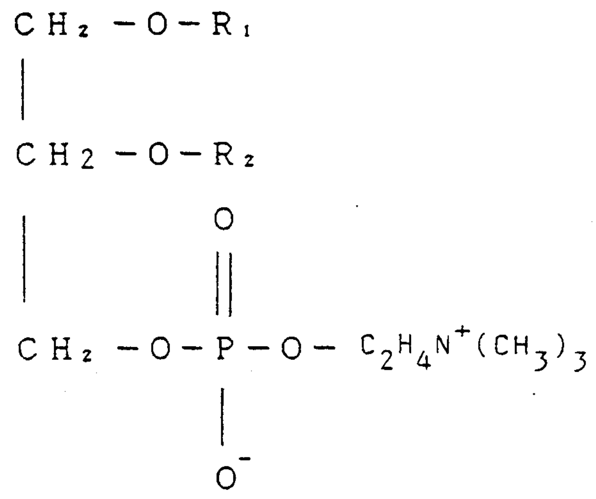

wherein $R_1$ and $R_2$ are as defined in claim 1, with a nucleoside of formula $N_s$-OH wherein $N_s$ is as defined in claim 1 in a solvent in the presence of phospholipase D-P; and, if desired, converting the resultant compound of formula (I) into a pharmacologically acceptable salt thereof.

**8.** A pharmaceutical composition comprising a nucleoside-phospholipid conjugate of formula (I) as defined in claim 1 or a pharmacologically acceptable salt thereof and a pharmacologically acceptable carrier or diluent.

**Revendications**

**1.** Conjugué nucléoside-phospholipide de la formule (I) :

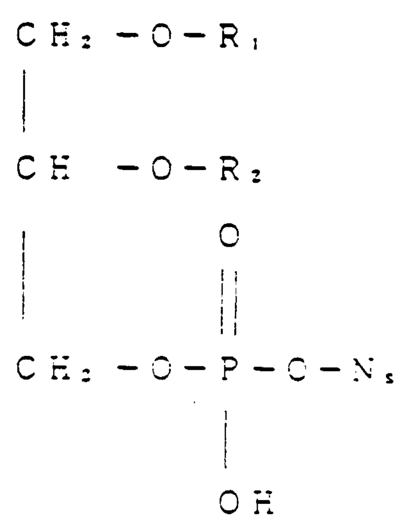

dans laquelle $R_1$ est un acyle aliphatique à longue chaîne à 14 à 24 atomes de carbone, un alcoyle aliphatique à 1 à 24 atomes de carbone, ou un alcényle ou un alcyne aliphatique à 2 à 24 atomes de carbone, $R_2$ est un acyle aliphatique à 1 à 10 atomes de carbone, un alcoyle aliphatique à 1 à 24 atomes de carbone, ou un alcényle ou un alcyne aliphatique à 2 à 24 atomes de carbone, et $N_s$ est un résidu de 5-fluorouridine-5'-yle, néoplanocine A-6'-yle ou aribinosyl-5-fluorocytosine-5'-yle, et leurs sels pharmacologiquement acceptables.

**2.** Composé selon la revendication 1, dans lequel $R_1$ est un acyle aliphatique à longue chaîne à 16 à 18 atomes de carbone et $R_2$ est un acyle aliphatique à 1 à 10 atomes de carbone.

**3.** Composé selon la revendication 1, dans lequel $R_1$ est un acyle aliphatique à longue chaîne à 16 à 18 atomes de carbone et $R_2$ est un alcoyle aliphatique à 1 à 10 atomes de carbone.

**4.** Composé selon la revendication 1, dans lequel $R_1$ et $R_2$ sont tous deux un alcoyle aliphatique à 1 à 24 atomes de carbone.

**5.** Composé selon la revendication 1, dans lequel $R_1$ est un groupe : palmitoyle, cétyle, stéaryle, oléyle, stéaroyle ou 9-octodécényle, et $R_2$ est un groupe : décanoyle, octanoyle, hexanoyle, butanoyle, acétyle, cétyle, stéaryle, oléyle, octyle, 9-octadécényle, méthyle ou hexyle.

**6.** Composé selon la revendication 1, dans lequel le conjugué nucléoside-phospholipide est un composé de type éther.

**7.** Procédé pour préparer un conjugué nucléoside-phospholipide de la formule (I) comme défini dans la revendication 1 ou un de ses sels pharmacologiquement acceptables, lequel procédé consiste à faire réagir un dérivé de glycérophospholipide de la formule (II) :

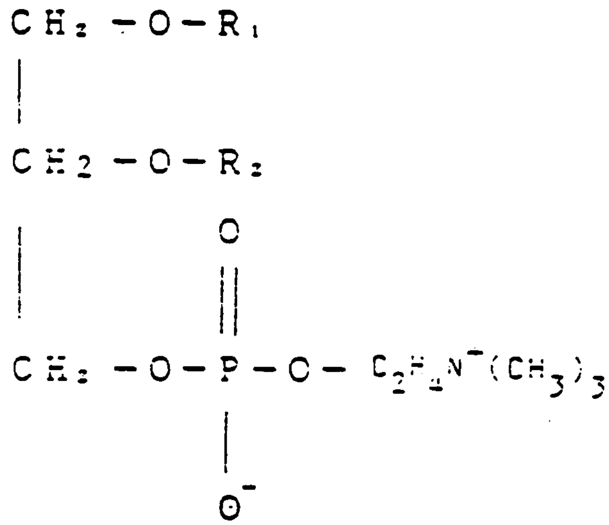

dans laquelle $R_1$ et $R_2$ sont comme définis dans la revendication 1, avec un nucléoside de la formule $N_s$-OH, dans laquelle $N_s$ est comme défini dans la revendication 1, dans un solvant en la présence de phospholipase D-P; et, si on le désire, à transformer le composé résultant de la formule (I) en un de ses sels pharmacologiquement acceptables.

**8.** Composition pharmaceutique comprenant un conjugué nucléoside-phospholipide de la formule (I) comme défini dans la revendication 1 ou un de ses sels pharmacologiquement acceptables, et un véhicule ou un diluant pharmacologiquement acceptable.

## Patentansprüche

1. Nukleosid-Phospholipid-Konjugat der allgemeinen Formel (I)

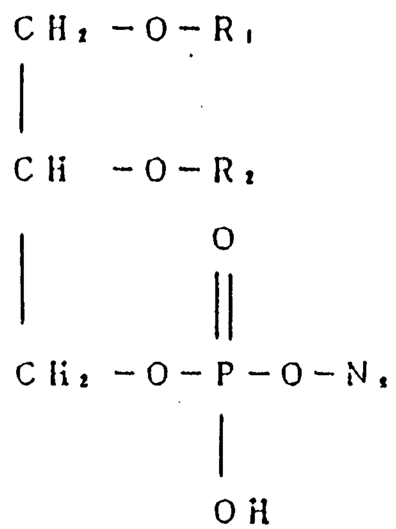

worin $R_1$ Für einen $C_{14-24}$-langkettigen aliphatischen Acylrest, einen $C_{1-24}$-aliphatischen Alkylrest oder $C_{2-24}$-aliphatischen Alkenyl- oder Alkinylrest steht, $R_2$ ein $C_{1-10}$-aliphatischer Acylrest, $C_{1-24}$-aliphatischer Alkylrest oder ein $C_{2-24}$-aliphatischer Alkenyl- oder Alkinylrest ist und $N_s$ einen 5-Fluoruridin-5'-ylrest, Neplanocin-A-6'-ylrest oder Aribinosyl-5-fluorcytosin-5'-ylrest darstellt, und dessen pharmakologisch verträgliche Salze.

2. Verbindung nach Anspruch 1, worin $R_1$ ein $C_{16-18}$-langkettiger aliphatischer Acylrest ist und $R_2$ einen $C_{1-10}$-aliphatischen Acylrest bedeutet.

3. Verbindung nach Anspruch 1, worin $R_1$ ein $C_{16-18}$-langkettiger aliphatischer Acylrest ist und $R_2$ einen $C_{1-10}$-aliphatischen Alkylrest darstellt.

4. Verbindung nach Anspruch 1, worin $R_1$ und $R_2$ beide $C_{1-24}$-aliphatische Alkylreste bedeuten.

5. Verbindung nach Anspruch 1, worin $R_1$ für Palmitoyl, Cetyl, Stearyl, Oleyl, Stearoyl oder 9-Octodecenyl steht und $R_2$ Decanoyl, Octanoyl, Hexanoyl, Butanoyl, Acetyl, Cetyl, Stearyl, Oleyl, Octyl, 9-Octadecenyl, Methyl oder Hexyl bedeutet.

6. Verbindung nach Anspruch 1, worin das Nukleosid-Phospholipid-Konjugat eine Verbindung vom Ethertyp ist.

7. Verfahren zur Herstellung eines Nukleosid-Phospholipid-Konjugats der allgemeinen Formel (I) gemäß Definition in Anspruch 1 oder eines pharmakologisch verträglichen Salzes davon, wobei ein Glycerophospholipid-Derivat der allgemeinen Formel (II)

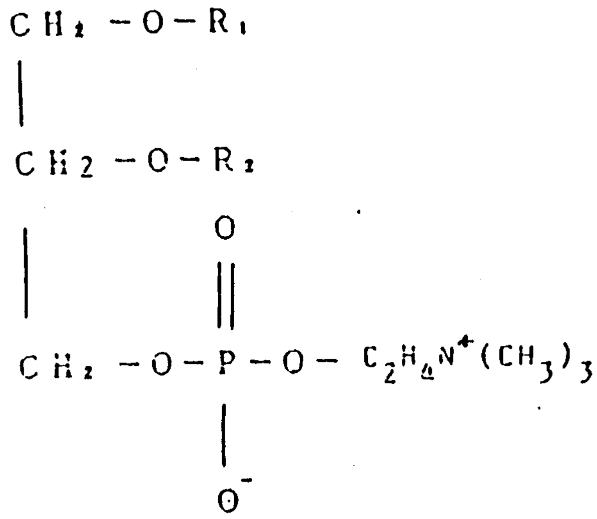

worin $R_1$ und $R_2$ der Definition in Anspruch 1 entsprechen, mit einem Nukleosid der Formel $N_s$-OH, worin $N_s$ der Definition in Anspruch 1 entspricht, in einem Lösungsmittel in Gegenwart von Phospholipase D-P umgesetzt wird, und gegebenenfalls die erhaltene Verbindung der Formel (I) in ein pharmakologisch verträgliches Salz davon überfuhrt wird.

**8.** Pharmazeutische Zusammensetzung, welche ein Nukleosid-Phospholipid-Konjugat der Formel (I) gemäß Definition im Anspruch 1 oder ein pharmakologisch verträgliches Salz davon enthält und einen pharmakologisch verträglichen Träger oder ein Verdünnungsmittel.